# EUROPEAN PATENT SPECIFICATION

(11) **EP 1 340 479 B1**
(45) Date of publication and mention of the grant of the patent: **02.05.2012**
(21) Application number: 03075515.1
(22) Date of filing: 24.02.2003
(51) Int. Cl.: C08J 5/18, A61F 13/15, B29C 47/00

(54) **Process for producing a biodegradable film for absorbent sanitary articles and nappies containing it**
Verfahren zur Herstellung eines biologisch abbaubaren Films für absorbierende Hygieneartikel und diesen enthaltende Windeln
Procédé de production d'un film biodégradable pour des articles d'hygiene et des couches le contenant

(30) Priority: 27.02.2002 IT MI20020386
(43) Date of publication of application: 03.09.2003
(73) Proprietor: Corman S.p.A., 20084 Lacchiarella MI (IT)
(72) Inventor: Oldani, Osvaldo, 34013 Duino Aurisina (Trieste) (IT); Tesolin, Laura, 33170 Pordenone (IT)
(74) Representative: Cicogna, Franco

(56) References cited:
- WO-A-00/58070
- WO-A-94/00163
- US-A- 5 262 458
- US-A- 5 366 782
- US-A- 5 422 387
- US-A- 6 027 787
- US-A- 6 127 512
- US-B1- 6 248 097
- T0480/98

## Description

### BACKGROUND OF THE INVENTION

The present invention relates to a process for producing a biodegradable film for absorbent sanitary articles and nappies containing it.

In particular, the present invention relates to a process for producing a biodegradable film which is impermeable to fluids and which can be used as an external wrapping for disposable nappies.

It is known that a wide variety and numerous types of sanitary articles for the absorption of body fluids are available on the market.

The disposable use of these absorbent articles together with their progressively increasing diffusion has created, in the last few years, problems relating to their disposal.

The absorbent sanitary articles currently on the market have a biodegradability of about 40% of their components as they are mainly made of synthetic plastic materials.

For example, children's nappies incorporate an external film made of non-biodegradable plastic on which non-woven fabrics normally made of polypropylene, polyester, adhesive polypropylene or polyester tapes, polypropylene elastic bands, polyurethane elastic threads, hot-melt glues and super-absorbent polymers made of sodium polyacrylate mixed with natural cellulose fibres, are supported. A nappy of this type, having an average weight of 50 g normally incorporates cellulose as sole biodegradable material, in a weight of about 20 g. Attempts made at substituting the plastic materials currently used in nappies with other biodegradable materials have had limited success due to the difficulty in finding a biodegradable product which is also impermeable to body fluids.

The documents US-A-5 262 458, WO 94/00163 and US-A-5 422 387 substantially disclose the preamble of claim 1.

### SUMMARY OF THE INVENTION

One of the general objectives of the present invention is to provide a process for producing a highly biodegradable film suitable as a wrapping for containing body liquids for absorbent sanitary articles which has high mechanical resistance characteristics, is particularly soft to touch and has almost no surface roughness.

A further objective of the present invention consists in providing a process for the production of disposable absorbent articles with an increased biodegradability, which does not involve high production costs.

Last but not least, an objective of the present invention consists in providing a process for producing high comfort disposable nappies, having improved biodegradability and/or composting characteristics.

In view of these objectives and others which will appear evident from the following description, a first aspect of the present invention relates to a process for the production of a film which is particularly suitable for being used as a containing layer of body fluids in absorbent articles, comprising the extrusion by means of bubble blowing of a biodegradable thermoplastic material.

The term biodegradable thermoplastic material refers to bioplastics deriving from natural raw materials. These thermoplastic materials, deriving from natural raw materials such as maize starch, maintain the same characteristics of use in absorbent articles as plastic materials traditionally used, even though they are substantially biodegradable. A typical example of a bioplastic is the material called Mater-Bi.

The film produced with the process of the invention is obtained by coupling two sheets of the bubble formed during the blowing operation. The coupling of the opposite surfaces of the bubble, made of biodegradable thermoplastic material, produces a film which is soft to the touch and is almost completely free of surface roughness.

The process of the invention allows the production of a film or coupled product made of biodegradable thermoplastic material, impermeable to body exudates or liquids eliminated by the organism and permeable to water vapour.

Furthermore, the coupling of the opposite surfaces of the bubble blown during extrusion gives the film high mechanical resistance characteristics summarized in Table I below, which make it particularly suitable for being used as an external wrapping for containing liquids in absorbent sanitary articles.

| Mechanical characteristics | Measurement Unit | Method | Range of values | Preferred range |
|---|---|---|---|---|
| MD elongation 5% | N/Inch | ASTM D882-95 | 0.4-0.85 | 0.55-0.65 |
| MD elongation 10% | N/Inch | ASTM D882-95 | 2-7 | 4-5 |
| MD Ultimate tensile strength | N/lnch | ASTM D882-95 | 21-35 | 27-29 |
| MD ultimate elongation | N/Inch | ASTM D882-95 | 130-360 | 210-250 |
| CD elongation 5% | N/lnch | ASTM D882-95 | 0.8-3.6 | 2-2.4 |
| CD elongation 10% | N/lnch | ASTM D882-95 | 1.7-4.7 | 3-3.4 |
| CD Ultimate tensile strength | N/inch | ASTM D882-95 | 7-16 | 11-12 |
| CD elongation | N/Inch | ASTM D882-95 | 500-900 | 650-750 |
| Friction coefficient | | ASTM D1894-94 | 0.4-0.85 | 0.6-0.65 |

The MD and CD data refer to the stretching values of the materials in the machine direction and transversal to the machine, respectively. The elongation data in the machine direction (MD) indicated above show that although the bioplastic film of the invention does not have high stretch properties, it has a low breakage susceptibility as the force necessary for breaking it ranges from 21 to 35 Newton.

According to an embodiment of the process of the invention, the extrusion phase is effected at a temperature close to the liquefaction temperature of the biodegradable thermoplastic material. Conveniently, the liquefaction process takes place with an increasing temperature gradient until reaching the melting point at which the extrusion takes place through an extrusion head by the insufflation of air to form a bubble.

The extrusion temperature preferably ranges from 150-160°C and more preferably is close to 155±3°C.

The air supplied in the swelling phase, which conveniently has a temperature ranging from 140-150°C and is preferably close to about 145°C, allows a suitable thickness or gram-surface ratio to be conferred to the end-film.

The pressure of the blown air advantageously ranges from 2 to 4 atmospheres.

The bubble produced is then conveniently pressed, using rolls, so as to form an overlapping of two sheets made of biodegradable thermoplastic material so that any possible imperfections or defects present on any of the two layers are flattened during the coupling phase with the other biodegradable polymeric layer.

According to this embodiment, the resulting film comprises two coupled bioplastic layers, is substantially without visible surface defects and has high mechanical resistance characteristics.

According to an embodiment, during the pressing phase, the bioplastic film is debossed and a crown treatment is optionally effected.

During the extrusion phase, the bioplastic film is conveniently stretched to give it softness making it particularly suitable for use in sanitary articles.

According to another preferred embodiment, the stretching is carried out in a longitudinal direction so as to reduce part of the elongation, causing a reduction in the extent of freedom of the film, thus making it more stable in the winding direction when it is processed in the production lines of sanitary articles. In particular, in the production of nappies, the film can be moderately elongated in the machine direction to allow a correct adhesion of all the other components of the nappy.

The film produced with the process of the invention conveniently has a gram-surface ratio within the range of 18-27.5 g/m² and preferably ranging from 22 to 26 g/m².

Another aspect of the present invention relates to an absorbent sanitary article comprising a biodegradable film produced with the process described above.

The article according to the invention conveniently comprises an internal layer permeable to liquids to be applied close to the pubic region of a person, an external biodegradable film produced with the process described above and an intermediate absorbent layer, suitable for absorbing liquids and exudates of the human body.

The permeable internal layer and the external biodegradable film are preferably firmly coupled by means of a glue of the hot melt type.

According to an embodiment, the absorbent article of the invention also has a barrier system suitable for substantially limiting the leakage of body exudates and/or means for its adherence to the body once applied.

The term sanitary absorbent article refers to articles which, when positioned in contact with or close to the pubic-sacral region of a person, absorb and withhold various body fluids or exudates released by the body. These articles are generally conceived for being thrown away after single use. An article of this kind can be worn by small children or incontinent adults, after being positioned and fixed between the user's legs. The term sanitary absorbent article consequently comprises children's nappies and protective towels for incontinent adults.

### BRIEF DESCRIPTION OF THE DRAWINGS

The characteristics and advantages of a sanitary absorbent article according to the present invention will appear more evident from the following illustrative but non-limiting description, referring to the enclosed schematic drawings in which:
- figure 1 shows an exploded view of an embodiment of a nappy produced according to a process of the invention;
- figure 2 shows a overhead view of the internal surface of a biodegradable film obtained according to an embodiment of the process of the invention.

### DESCRIPTION OF THE PREFERRED EMBODIMENT

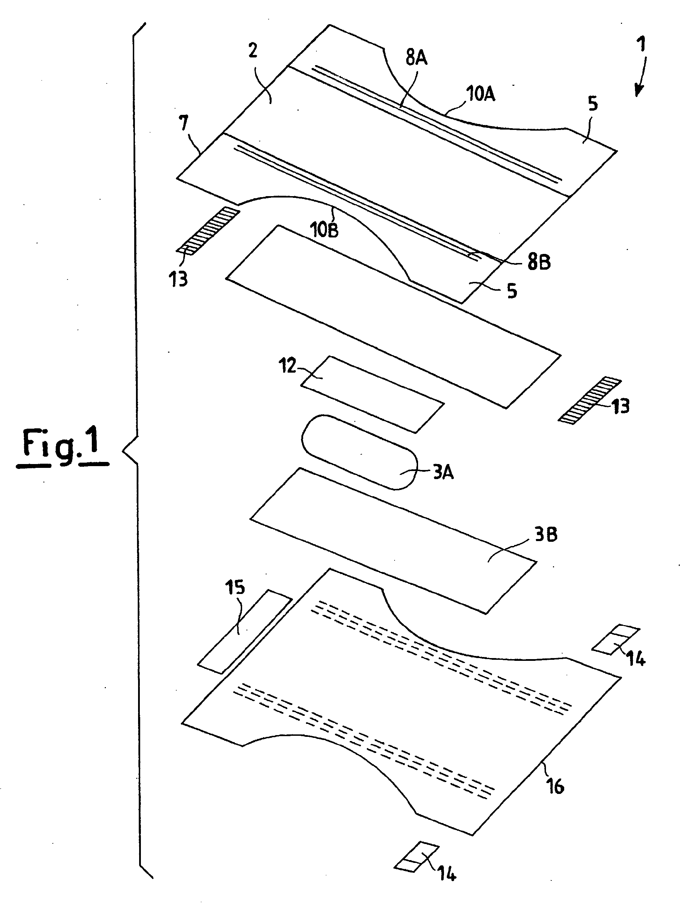

With reference to the enclosed Figure 1, it can be observed that the upper permeable layer 2 of the nappy 1, has almost the same dimensions as the external lower layer 16 so as to create an exact overlapping of the two external layers of the nappy 1.

The perimeter of the nappy 1 is extended and is bordered from the upper end 7 to the lower end 6, by the side flaps 5 and by the two leg openings 10A and 10B.

The upper layer 2 of the nappy is made of a film permeable to body fluids which remains for the most part in contact with the user's skin. The side flaps 5 are the portions close to the upper end 7 and lower end 6 which overlap when the nappy 1 is being worn by the user and consequently when the nappy 1 is attached to the user's sides.

The internal absorbing layer 3, positioned between the upper layer 2 and biodegradable lower layer 16 of the invention, can have various shapes and dimensions proportional to the size of the nappy. This absorbing layer 3, which is suitable for absorbing and withholding body liquids and certain exudates, can, for example, be thin and pressed or bulky and conformable. It can be made of a variety of materials capable of withholding and absorbing urine commonly used in disposable absorbent articles. Examples of these absorbing materials comprise cellulose fluff, super-absorbent polymers, gelifying absorbing material, cotton or equivalent absorbing materials. The concentration and weight of the absorbing material varies from size to size to ensure that the expected quantity of liquids or exudates for the specific size is absorbed and withheld. The absorbing layer 3 can include a single or double layer. In particular, the nappy 1 has a double absorbing layer indicated as 3A and 3B. The upper absorbing layer 3A has the function of rapidly collecting the body liquids. Said upper absorbing layer 3A generally comprises fibres of hydrophilic material, without or with limited quantities of gelifying absorbent material.

This absorbing layer 3A is normally positioned in the area where there is the greatest necessity, which corresponds to the central-front area of the nappy, between the upper cut end 7 and the lower cut end 6. The lower absorbing layer 3B has the function of absorbing and withholding the liquids filtered by the upper layer 3A. The lower layer 3B, which generally has greater dimensions than the upper absorbing layer 3A, conveniently extends for almost the whole length of the nappy 1, from the upper end 7 to the lower end 6, compatibly with the possibility of sealing the nappy near the end. The absorbing layer can be positioned in continuous along the length of the nappy or intermittently so as to create areas with a greater concentration of absorbing material.

A thin collecting layer 12, also made of natural or synthetic transpirant material, and a lining layer 11 made of transpirant fabric, can conveniently be superimposed on said absorbing layers 3A and 3B.

As the upper layer 2 of the nappy 1 is in close contact with the user's skin, it must absorb the waste body liquids and is made of materials permeable to liquids, which are soft and non-irritating to allow their rapid passage to the underlying absorbing layer(s) 3. The upper filtering layer 2 can be made of natural fibres such as cellulose, cotton, viscose and other similar materials, synthetic fibres such as polypropylene, polyester and other similar products or a mixture of the two types of fibres.

The upper transpirant layer 2 advantageously comprises at least one hydrophobic portion which forms a barrier for feces in order to effectively prevent their leakage from the nappy. For example, said hydrophobic portion comprises two strips or barriers 8A and 8B made of hydrophobic material arranged longitudinally to the nappy 1, each close to the leg openings 10A and 10B of the nappy 1. These fecal barriers 8A, 8B conveniently comprise elastic elements, generally elastic filaments made of natural rubber or synthetic elastic materials such as polyurethane, suitable for the adherence of the nappy 1 to the user's legs and preventing the leakage of feces or liquids. According to another embodiment, said upper layer 2 of the nappy 1 comprises a hydrophilic portion which extends to the central part only so that it is situated above the absorbing layer 3. For example, said hydrophilic portion substantially extends longitudinally along the whole length of the upper surface 2 of the nappy and is bordered at the side by the presence of elastic barriers 8. The hydrophobic portion in this case extends along the side flaps and close to the leg openings 10A and 10B.

The nappy 1 of the invention can also be provided with elasticizing elements 9A and 9B situated close to the leg openings 10A and 10B respectively. These elastic elements 9A and 9B, advantageously comprise a series of stretched elastic threads capable of gripping the user's legs when the nappy 1 is applied. The nappy 1 can also comprise elastic bands 13A and 13B positioned near the ends 6 and 7 of the nappy 1 to ensure a firmer grip when applied to a person.

The outer layer 16 of the nappy 1 comprises a film made of biodegradable thermoplastic material, of the type described above.

This biodegradable film, is advantageously made of a bioplastic material such as Mater-Bi, is impermeable to body liquids and exudates thus preventing the exudates absorbed by the central absorbing layer 3 from fouling linen, sheets, clothes or other articles which come in contact with the nappy. As said outer layer 3 is permeable to the water vapour mainly formed in the absorbing layer 3, it prevents humidity from accumulating in the nappy 1 and irritating the skin or jeopardizing the physiological functioning of skin perspiration also in newly-born babies. In addition, the high biodegradability of the lower layer 16 considerably limits disposal problems linked to the use of disposable nappies.

The nappies of the invention have a more favourable environmental impact with respect to traditional nappies, as they do not significantly contribute to the impoverishment of natural substances.

Furthermore, the use of bioplastics for their production also allows a reduction in the degradation times of the cellulose absorbing component inside, thus creating a synergy in the biodegradability of the article.

According to an embodiment of the invention, said lower layer 16 of the nappy 1 is made opaque by the addition of suitable biodegradable pigments of a natural origin or with Mater Batch which make it appear like an item of clothing.

According to another aspect, the present invention relates to a method for producing an absorbent sanitary article of the type described above, which comprises the coupling of at least one portion of a film made of biodegradable thermoplastic material with at least one portion of a film permeable to liquids wherein said coupling is effected by the spreading of a hot melt glue at a temperature which does not tear or substantially jeopardize the integrity of said biodegradable film.

According to a preferred embodiment, said hot melt glue is a glue based on thermoplastic rubber.

The Applicant has verified that in order to limit the tearing of said biodegradable film during the production of the nappy, it is preferable to avoid the use of glues based on ethyl-vinyl-acetate (EVA) which have high application temperatures, normally within the range of 170 to 200°C.

The application of the hot melt glue is advantageously effected by contact spreading on a flat head whereas the spray application is limited to fixing the elastic threads close to the leg openings of the nappy.

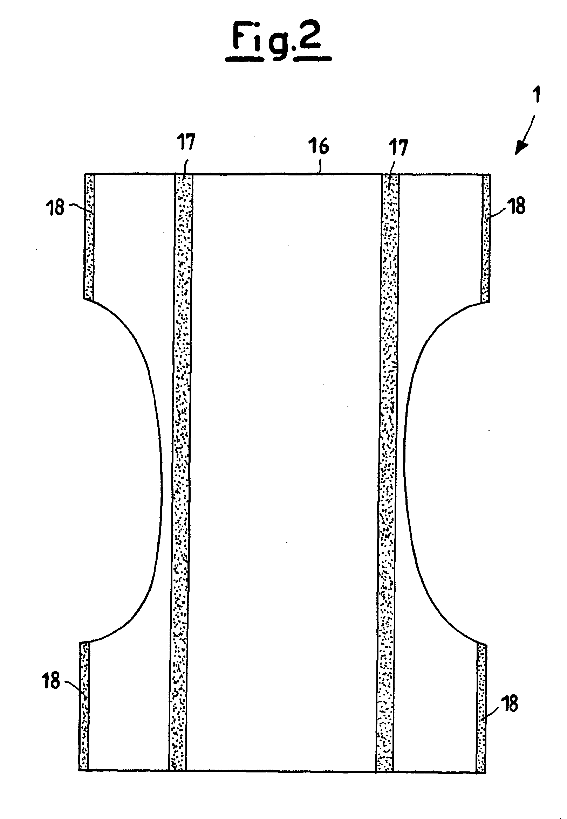

With reference to the enclosed Figure 2, this illustrates an embodiment of an external bioplastic film 16, obtained with the method of the invention, of a nappy for children or newly-born babies. According to this embodiment, the application of the hot melt glue is effected along the surface of the bioplastic film 16 except for two longitudinal shaded side strips 17, destined for fixing the elastic elements of the leg openings, and the side ends 18 of the flaps 5.

The spreading of the glue onto the film 16 is effected by monitoring the gluing temperature so that it remains within the range of 130°±5°C to ensure that the film is kept intact.

The gram-surface ratio of the hot melt glue is conveniently equal to about 4 g/m² ± 0.8 g/m² and the application of the elastic elements is effected with a spray system, setting the temperature of the glue to be sprayed at about 140-155°C, with a pressure within the range of 3-4 atm. and a temperature of the air of about 150-165°C. Said elastic elements are preferably elastic threads applied close to the two leg openings, for example 3 per side, at a distance of 3 to 5 mm from each other.

## Claims

1. A process for making a biodegradable film to be used as a containment layer of liquids eliminated from a human body in absorbent sanitary articles, said process comprising a step of bubble-blowing extruding a biodegradable thermoplastic material to provide a biodegradable thermoplastic material bubble, **characterized in that** said process comprises a further step of roll pressing said thermoplastic bubble so as to couple opposite surfaces of said bubble to form said biodegradable film.

2. A process according to claim 1, **characterized in that** said biodegradable film has a gram-surface ratio within the range of 18-27.5 g/m².

3. A process according to claims 1-2, **characterized in that** in said extruding step is used blowing air having a pressure from 2 to 4 atmospheres.

4. A process according to claims 1-3, **characterized in that** said extrusion step is performed at a temperature close to a liquefaction temperature of said biodegradable thermoplastic material.

5. A process according to claims 1-4, **characterized in that** in said roll-pressing step a debossing and/or crown treatment is performed.

6. A process according to claims 1-5, **characterized in that** said process further comprises an end step of stretching said film.

7. A biodegradable film made by a process according to claim 1.

8. An absorbent sanitary article comprising an internal layer permeable to human body to be applied close to a pubic region of a person, a central layer absorbing body liquids and exudates and an outer body liquids holding layer, **characterized in that** said outer holding layer comprises a biodegradable film according to claim 7.

9. An article according to claim 8, **characterized in that** said article is a children or newly-born baby nappy.

10. An article according to claim 8 or 9, **characterized in that** said article also comprises a barrier system for substantially limiting a leakage of feces and liquids when worn.

11. An article according to claims 8-10, **characterized in that** said article also comprises a closing system positioned on outer flaps and elasticized fecal barriers close to two side leg openings.

12. An article according to claim 8, **characterized in that** said central absorbing layer comprises a biodegradable absorbing material.

13. An article according to claim 12, **characterized in that** said biodegradable absorbing material comprises cellulose fluff, cotton, natural absorbing fibers and their mixtures.

14. An article according to claim 8, **characterized in that** said article also comprises at least a liquid permeable layer arranged above said central absorbing layer.

15. An article according to claims 8 and 11, **characterized in that** elastic threads are applied on an internal surface of said outer layer and close to said leg openings to prevent a body liquid leakage.

16. An article according to claim 8, **characterized in that** said liquid impermeable outer layer comprises biodegradable natural pigments or Mater Batch to provide said article with an appearance similar to a clothing item.

17. A process for making an absorbent sanitary biodegradable article, said process comprising the steps of coupling at least a portion of a biodegradable film according to claim 7 to at least a portion of liquid permeable film, **characterized in that** said coupling is carried out by spreading a hot melt glue at a temperature lower than a liquefaction temperature of said films.

18. A process according to claim 17, **characterized in that** said article is a children or newly-born baby nappy.

19. A process according to claim 17, **characterized in that** said hot melt glue is a thermoplastic rubber based hot melt glue.

20. A process according to claim 17, **characterized in that** said spreading is performed on a flat head at a temperature from 125-135°C, thereby limiting a tearing of said biodegradable film.

21. A process according to claim 17, **characterized in that** said hot melt glue has a gram-surface ratio within a range of 4 g/m² ± 0.8 g/m².

22. A process according to claim 17, **characterized in that** said process further comprises a step of applying elastic elements to said article by spray applying a glue on said article.

23. A process according to claims 17-22, **characterized in that** said process further comprises a step of applying at least an intermediate absorbent layer to said coupled films.

## Patentansprüche

1. Verfahren zum Herstellen einer biologisch abbaubaren Folie zur Verwendung als Rückhalteschicht für Flüssigkeiten, die aus einem menschlichen Körper ausgeschieden werden, in saugfähigen Hygieneartikeln, wobei das Verfahren einen Schritt der Blasenextrusion eines biologisch abbaubaren thermoplastischen Materials umfasst, um ein biologisch abbaubares, geblasenes thermoplastisches Material vorzusehen, **dadurch gekennzeichnet, dass** das Verfahren einen weiteren Schritt des Walzenpressens von dem geblasenen thermoplastischen Material umfasst, um entgegengesetzte Oberflächen von dem geblasenen Material für die Bildung der biologisch abbaubaren Folie zu verbinden.

2. Verfahren nach Anspruch 1, **dadurch gekennzeichnet, dass** die biologisch abbaubare Folie ein Gramm-Oberflächen-Verhältnis innerhalb eines Bereiches von 18 - 27,5 g/m² aufweist.

3. Verfahren nach den Ansprüchen 1 bis 2, **dadurch gekennzeichnet, dass** in dem Extrusionsschritt das Blasen von Luft mit einem Druck von 2 bis 4 Atmosphären verwendet wird.

4. Verfahren nach den Ansprüchen 1 bis 3, **dadurch gekennzeichnet, dass** der Extrusionsschritt bei einer Temperatur durchgeführt wird, die nahe bei der Verflüssigungstemperatur des biologisch abbaubaren thermoplastischen Materials liegt.

5. Verfahren nach den Ansprüchen 1 bis 4, **dadurch gekennzeichnet, dass** in dem Schritt des Walzenpressens eine Tiefprägung und/oder Corona-Behandlung durchgeführt wird.

6. Verfahren nach den Ansprüchen 1 bis 5, **dadurch gekennzeichnet, dass** das Verfahren ferner einen Endschritt des Streckens der Folie umfasst.

7. Biologisch abbaubare Folie, die nach einem Verfahren gemäß Anspruch 1 hergestellt ist.

8. Saugfähiger Hygieneartikel, der eine Innenschicht, die zum menschlichen Körper hin durchlässig ist, welche in der Nähe der Schamgegend einer Person eingesetzt wird, eine zentrale Schicht, die Körperflüssigkeiten und Sekrete absorbiert, und eine die Körperflüssigkeiten zurückhaltende Außenschicht aufweist, **dadurch gekennzeichnet, dass** die äußere Rückhalteschicht eine biologisch abbaubare Folie nach Anspruch 7 aufweist.

9. Hygieneartikel nach Anspruch 8, **dadurch gekennzeichnet, dass** der Hygieneartikel eine Windel für Kinder oder neugeborene Babys ist.

10. Hygieneartikel nach Anspruch 8 oder 9, **dadurch gekennzeichnet, dass** der Hygieneartikel ebenfalls ein Rückhaltesystem umfasst, um im Wesentlichen ein Auslaufen von Fäkalien und Flüssigkeiten beim Tragen einzuschränken.

11. Hygieneartikel nach den Ansprüchen 8 bis 10, **dadurch gekennzeichnet, dass** der Hygieneartikel ebenfalls ein Verschlusssystem, das an den äußeren Laschen angeordnet ist, und elastisch eingerichtete Fäkaliensperren nahe bei den beiden seitlichen Schenkelöffnungen aufweist.

12. Hygieneartikel nach Anspruch 8, **dadurch gekennzeichnet, dass** die zentrale absorbierende Schicht ein biologisch abbaubares Material umfasst.

13. Hygieneartikel nach Anspruch 12, **dadurch gekennzeichnet, dass** das biologisch abbaubare, absorbierende Material Zelluloseflocken, Baumwolle, natürliche absorbierende Fasern und deren Mischungen umfasst.

14. Hygieneartikel nach Anspruch 8, **dadurch gekennzeichnet, dass** der Hygieneartikel ebenfalls mindestens eine für Flüssigkeiten durchlässige Schicht aufweist, die über der zentralen absorbierenden Schicht angeordnet ist.

15. Hygieneartikel nach den Ansprüchen 8 und 11, **dadurch gekennzeichnet, dass** elastische Fäden auf der Innenfläche der äußeren Schicht und nahe bei den Schenkelöffnungen angebracht sind, um das Auslaufen von Körperflüssigkeiten zu verhindern.

16. Hygieneartikel nach Anspruch 8, **dadurch gekennzeichnet, dass** die äußere Schicht, die für Flüssigkeiten undurchlässig ist, biologisch abbaubare natürliche Pigmente oder Mater-Batch aufweist, um den Hygieneartikeln ein Aussehen zu verleihen, das einem Kleidungsstück ähnlich ist.

17. Verfahren zur Herstellung eines absorbierenden, biologisch abbaubaren Hygieneartikels, wobei das Verfahren die Schritte des Verbindens von mindestens einem Teilbereich der biologisch abbaubaren Folie nach Anspruch 7 mit mindestens einem Teilbereich der für Flüssigkeiten durchlässigen Folie, **dadurch gekennzeichnet, dass** das Verbinden durchgeführt wird durch das Verteilen von Heißschmelzklebstoff bei einer Temperatur, die niedriger ist als die Verflüssigungstemperatur der Folie.

18. Verfahren nach Anspruch 17, **dadurch gekennzeichnet, dass** der Hygieneartikel eine Windel für Kinder oder neugeborene Babys ist.

19. Verfahren nach Anspruch 17, **dadurch gekennzeichnet, dass** der Heißschmelzklebstoff ein Heißschmelzklebstoff auf der Basis von thermoplastischem Kautschuk ist.

20. Verfahren nach Anspruch 17, **dadurch gekennzeichnet, dass** das Aufbringen mit einem Flachkopf-Drucker bei einer Temperatur von 125 - 135 °C durchgeführt wird, wodurch ein Zerreißen der biologisch abbaubaren Folie eingeschränkt wird.

21. Verfahren nach Anspruch 17, **dadurch gekennzeichnet, dass** der Heißschmelzklebstoff ein Gramm-OberflächenVerhältnis innerhalb eines Bereiches von 4 g/m² ± 0,8 g/m² aufweist.

22. Verfahren nach Anspruch 17, **dadurch gekennzeichnet, dass** das Verfahren ferner einen Schritt des Aufbringens elastischer Elemente auf dem Hygieneartikel mittels Sprühauftragung eines Klebers auf den Hygieneartikel.

23. Verfahren nach den Ansprüchen 17 bis 22, **dadurch gekennzeichnet, dass** das Verfahren ferner einen Schritt des Aufbringens von mindestens einer absorbierenden Zwischenschicht zwischen die verbundenen Folien umfasst.

## Revendications

1. Procédé de fabrication d'un film biodégradable pour être utilisé comme couche de confinement des fluides éliminés par un corps humain dans des articles d'hygiène absorbants, ledit procédé comprenant une étape d'extrusion par soufflage d'air d'un matériau thermoplastique biodégradable afin de réaliser une bulle de matériau thermoplastique biodégradable, **caractérisé en ce que** le procédé comprend une autre étape de passage à la presse à cylindres de ladite bulle thermoplastique de manière à assembler les surfaces opposées de ladite bulle pour former ledit film biodégradable.

2. Procédé selon la revendication 1, **caractérisé en ce que** ledit film biodégradable a un rapport gramme/surface compris dans une fourchette de 18 g/m² à 27,5 g/m².

3. Procédé selon les revendications 1 à 2, **caractérisé en ce que**, au cours de ladite étape d'extrusion, on utilise de l'air soufflé à une pression comprise entre 2 et 4 atmosphères.

4. Procédé selon les revendications 1 à 3, **caractérisé en ce que** l'étape d'extrusion est réalisée à une température proche d'une température de liquéfaction dudit matériau thermoplastique biodégradable.

5. Procédé selon les revendications 1 à 4, **caractérisé en ce que**, pendant ladite étape de passage à la presse à cylindres, un dégaufrage et/ou un traitement à couronne est réalisé.

6. Procédé selon les revendications 1 à 5, **caractérisé en ce que** ledit procédé comprend en outre une étape finale d'étirage dudit film.

7. Film biodégradable fabriqué grâce à un procédé selon la revendication 1.

8. Article d'hygiène absorbant comprenant une couche interne perméable aux fluides corporels humains qui doit être appliquée près de la région pubienne d'une personne, une couche centrale absorbant les fluides et les exsudats corporels et une couche extérieure retenant les fluides corporels, **caractérisé en ce que** ladite couche extérieure de retenue comprend un film biodégradable selon la revendication 7.

9. Article selon la revendication 8, **caractérisé en ce que** ledit article est une couche pour enfants ou pour nouveaux nés.

10. Article selon les revendications 8 ou 9, **caractérisé en ce que** ledit article comprend également un système de barrière permettant de limiter sensiblement une fuite de selles et de liquides quand il est porté.

11. Article selon les revendications 8 à 10, **caractérisé en ce que** ledit article comprend également un système de fermeture placé sur les rabats extérieurs et des barrières fécales élastiques près des deux ouvertures latérales pour les jambes.

12. Article selon la revendication 8, **caractérisé en ce que** ladite couche absorbante centrale comprend un matériau absorbant biodégradable.

13. Article selon la revendication 12, **caractérisé en ce que** ledit matériau absorbant biodégradable comprend de la ouate de cellulose, du coton, des fibres naturelles absorbantes et des mélanges de ceux-ci.

14. Article selon la revendication 8, **caractérisé en ce que** ledit article comprend également au moins une couche perméable aux liquides agencée par dessus ladite couche absorbante centrale.

15. Article selon les revendications 8 et 11, **caractérisé en ce que** des fils élastiques sont appliqués sur une surface interne de ladite couche extérieure et près desdites ouvertures pour les jambes afin d'empêcher toute fuite de fluide corporel.

16. Article selon la revendication 8, **caractérisé en ce que** ladite couche extérieure imperméable aux liquides comprend des pigments naturels biodégradables ou du Mater Batch pour donner au dit article un aspect semblable à celui d'un vêtement.

17. Procédé de fabrication d'un article d'hygiène absorbant biodégradable, ledit procédé comprenant les étapes consistant à assembler au moins une partie d'un film biodégradable selon la revendication 7 à au moins une partie d'un film perméable aux liquides, **caractérisé en ce que** ledit assemblage est réalisé en étalant une colle thermofusible à une température inférieure à la température de liquéfaction desdits films.

18. Procédé selon la revendication 17, **caractérisé en ce que** ledit article est une couche pour enfants ou pour nouveaux nés.

19. Procédé selon la revendication 17, **caractérisé en ce que** ladite colle thermofusible est une colle thermofusible à base de caoutchouc thermoplastique.

20. Procédé selon la revendication 17, **caractérisé en ce que** ledit étalage est réalisé sur une tête plate à une température comprise entre 125 et 135 °C, ce qui limite ainsi un déchirement dudit film biodégradable.

21. Procédé selon la revendication 17, **caractérisé en ce que** ladite colle thermofusible a un rapport gramme/surface compris dans une fourchette de 4 g/m² +/- 0,8 g/m².

22. Procédé selon la revendication 17, **caractérisé en ce que** ledit procédé comprend en outre une étape consistant à appliquer lesdits éléments élastiques sur ledit article en appliquant par pulvérisation une colle sur ledit article.

23. Procédé selon les revendications 17 à 22, **caractérisé en ce que** ledit procédé comprend en outre une étape consistant à appliquer au moins une couche absorbante intermédiaire sur lesdits films assemblés.
